# EUROPEAN PATENT SPECIFICATION

(11) **EP 3 248 537 B1**
(45) Date of publication and mention of the grant of the patent: **25.09.2019**
(21) Application number: 15879122.8
(22) Date of filing: 22.01.2015
(51) Int. Cl.: A61B 1/31, A61B 8/00, A61B 1/303, A61B 1/06, A61B 8/12

(54) **PROCTOSCOPE**
PROKTOSKOP
RECTOSCOPE

(43) Date of publication of application: 29.11.2017
(73) Proprietor: AO NPF "BIOSS", Moscow, Zelenograd 124489 (RU)
(72) Inventor: ARSHINOV, Boris Viktorovich, Moscow Zelenograd 124460 (RU); BOLKHOVITIN, Sergey Nikolaevich, Moscow Zelenograd 124681 (RU); TSYBIN, Igor Mihaylovich, Moscow Zelenograd 124683 (RU)
(74) Representative: Viering, Jentschura & Partner mbB Patent- und Rechtsanwälte
(86) International application number: PCT/RU2015/000038
(87) International publication number: WO 2016/118041

(56) References cited:
- EP-A2- 1 234 539
- EP-A2- 1 234 539
- WO-A1-2004/004555
- WO-A1-2010/041280
- US-A- 5 570 692

## Description

### FIELD OF THE INVENTION

The device relates to instruments for medical examination and for diagnostic and treatment procedures directed to internal cavities (more particularly to a rectum) and conducted by visual inspection and by employing ultrasonic waves. The device (a proctoscope) is employed in coloproctology, in particular for diagnostics and treatment of haemorrhoids and is used in performing suture ligation of haemorrhoidal vessels conducted under the control by Doppler ultrasonography.

### BACKGROUND OF THE INVENTION

From the prior art, there is known a device for surgical treatment of prolapse, the device comprising: a hollow elongated housing which extends along the longitudinal axis, is adapted to be inserted into an opening and has a window limiting an intervention area and providing communication between an internal cavity of the elongated housing and a part of prolapse; and a mobile wall defining, together with the elongated housing, said window and slidably connected to the housing to enable opening and/or closing the window to attain configurations corresponding to its minimum and maximum length. The device is characterized in that it comprises locking means which locks the movable wall relative to the elongated housing and reliably maintains the window in the configuration of its minimum length, with keeping the wall in a closed position during insertion of the elongated housing into said opening (see RU 2480138 C2, April 04, 2013).

There is also known a device for surgical operations on haemorrhoidal prolapse, the device comprising a hollow divaricator extending in a prevailing direction along the longitudinal axis and insertable into patient's anal orifice. The divaricator has a window limiting the working area and providing communication between the interior of divaricator and a section of haemorrhoidal prolapse. The device further comprises means for opening and closing the window to facilitate changing the operation zone, said means for opening and closing the window comprising a mobile wall adapted to move from a closed position to an open position by a sliding movement of said movable wall towards the rear end of the divaricator. The window has a first portion extending substantially transversely to the longitudinal axis, and a second portion extending in parallel to the longitudinal axis, wherein the movable wall operates in the area of the second portion of the window for attaining its opening or closing (see RU 2408290 C2, March 03, 2010). This device has also a light source located in the hand grip or in an expanded part of a barrel and extending along the barrel.

There is further known a disposable device for surgical operations on the arteria haemorroidalis, the device comprising a retractor tube closed and rounded at the end with which it is inserted into the anal orifice. The retractor tube is provided upon its side surface with at least one examination window through which there appears the rectal mucosa upon which the operation for the ligature of the artery will be made. The device is provided with a hand grip integral with the tube's external mouth (preferably diverging), through which mouth it is possible to observe the tissue through said window and to insert into the tube the instruments required for a surgical operation, and with means to illuminate said window and the mucosa which appears through this window. Said illumination means comprises a light source fixed in removable manner inside the hand grip. The device further comprises, at the level of an area joining the hand grip with the outer edge of the retractor tube, light reflecting means for reflecting the light emitted by said source so as to illuminate with the reflected light the inner portion of the retractor tube and in particular said lateral window. The device is further characterized in that the retractor tube is coaxially arranged with the examination window, and an elongated linear internal chamber is placed in front of it. The chamber is separated by a partition formed integrally with the inner walls of the tube and the rear side of the window, and the whole structure is designed so that the chamber is open towards said external mouth of said tube, wherein an ultrasonic probe is inserted, with friction, into said chamber, which probe partially protrudes from the opening arranged longitudinally in a lateral wall of the retractor tube, so that the probe is engageable with the rectal mucous membrane (see RU 2308873 C2, October 27, 2007). This device also uses the light source which is located in the hand grip and does not provide sufficient illumination, while the probe is located in the area preceding the window intended for the mucosa examination.

An anoscope for ligating internal haemorrhoids has an outer tubular body with lateral haemorrhoid-receiving apertures coinciding with the normal location of internal haemorrhoids in a man, and a generally bullet-shaped head to close the distal end of the anoscope. A rotatable inner obturator has lateral apertures that register with the lateral apertures of the outer tubular body when the anoscope is open, and intermediate slats which occlude the lateral apertures when the anoscope is closed (see WO 2007/032776 A1, March 22, 2007). This device does not have an ultrasonic sensor, so that its diagnostic value is reduced.

The prior art solution closest to the claimed device is constituted by an arrangement for the ligation of arteries in hollow members. The arrangement comprises a hand grip and a tube which is placed in the rectum. The arrangement has a proximal region where the hand grip is located, and a distal region containing an ultrasonic probe for localizing an artery, as well as an opening providing access to the rectum surface. The device also has an illumination unit composed of a light guide which transmits light from the proximal end to the distal end of the unit. The tube is made of polycarbonate or polysulfone (see EP 1234539 A2, August 28, 2002). In the prior art arrangement, an ultrasonic device is placed in front of the suturing area, that is in front of the window for access to the rectum surface (when viewed from the surgeon's side), while the light is directed from the surgeon to the window. Such configuration can be insufficiently effective due to the remoteness of the light sources, which, in its turn, affects the quality of the manipulations to be performed.

### SUMMARY OF THE INVENTION

The object of this technical solution is to increase the usability and reliability of functioning of the device and to improve stability of an ultrasonic investigation while ensuring increased strength of the device and effectiveness of manufacture thereof.

This object is achieved by that the proctoscope comprising a translucent housing having an opening for access to the rectum surface and a housing head; an ultrasonic (US) sensor; a light source; and a hand grip, ***according to the proposed technical solution,*** further includes, in the bottom of the housing, a compartment having internal side walls, said compartment having disposed therein a plate or panel which has light emitting diodes (LEDs) arranged in the lower part thereof, along its length, and functioning as a light source, wherein said LEDs are arranged in such a way that the light flux therefrom is oriented primarily perpendicularly to the internal side walls of the compartment, while the ultrasonic sensor is accommodated in an upper part of said plate or panel, said sensor having an outlet to the outer surface of the housing in the terminal part thereof located between the opening and the housing head, and wherein the sensor is tilted towards the opening.

The plate or panel may be adapted to serve as a shield providing protection from a part of the light flux generated by the LEDs.

The terminal part of the housing may be divided by a partition wall from the remaining part of the housing.

The partition wall may have a bulge in its upper part, said bulge serving, on one side of said wall, as a support for the US sensor, wherein recesses serving as supports for a medical instrument are provided in the opposite side of said wall.

Two rounded recesses may be formed in said wall, said recesses being connected by a groove to ensure support for the medical instrument.

The housing head may be made removable and may be supplied with projecting guides having snap latches for fixedly attaching the head to the housing.

The housing head may be provided with projecting stops for fixedly attaching the US sensor.

The proctoscope may further comprise a removable insert supplied with skids and with locks and fixedly positioned in the housing partially overlapping the opening.

The terminal part of the plate or panel may be positioned in abutment inside the head.

The technical result consists in increasing accuracy of manipulations to be performed and in functional effectiveness of the device by improving the illumination of the target zone by appropriately manipulating the light source consisting of a plurality of LEDs arranged along the axis of the housing close to the target area such that a light flux is spread through the inner side walls inside the translucent housing and so does not create pronounced shadows. This results in making it possible to conduct the manipulations under the constant visual and ultrasonic control to produce, in the on-line mode, a clear, stable and reliable signal from a vessel in the manipulations area due to the inclined (or tilted) position of the ultrasonic sensor behind the opening inside which ligation of haemorrhoids vessels is performed, owing to that the area of location of vessels by the US sensor is closer to the ligation area. Moreover, the structure as the whole becomes more sturdy and reliable, better adapted for effective manufacture owing to that parts of the device may be manufactured separately and then assembled in a modular way. This is true in particular for mounting the light sources and the US sensor inside the housing.

When conducting medical treatment and diagnostic procedures, especially surgical manipulations, a modern safety level requires the widest possible use of disposable products, especially made of polymer materials.

It is pointed out in WO 2004/064624 (RU 2308873) that a device (a proctoscope) disclosed therein "for the reason that it incorporates the ultrasonic probe and houses the luminous source in its closed end, presents elevated production costs, so that it is not possible to propose the same as a disposable product, with all the drawbacks and the limitations deriving by this fact". In contrast to this prior art device, with the proposed device which contains the built-in ultrasonic sensor and the built-in light source, it becomes possible to eliminate this disadvantage.

The design of the proposed device greatly simplifies (due to the improved processability) the process of its manufacturing, thus making the device available for a single use and making it more convenient to employ.

### BRIEF DESCRIPTION OF DRAWINGS

The claimed technical solution will now be described in more detail with references to the appended drawings, which do not cover the entire scope of the invention but only illustrate a particular embodiment.

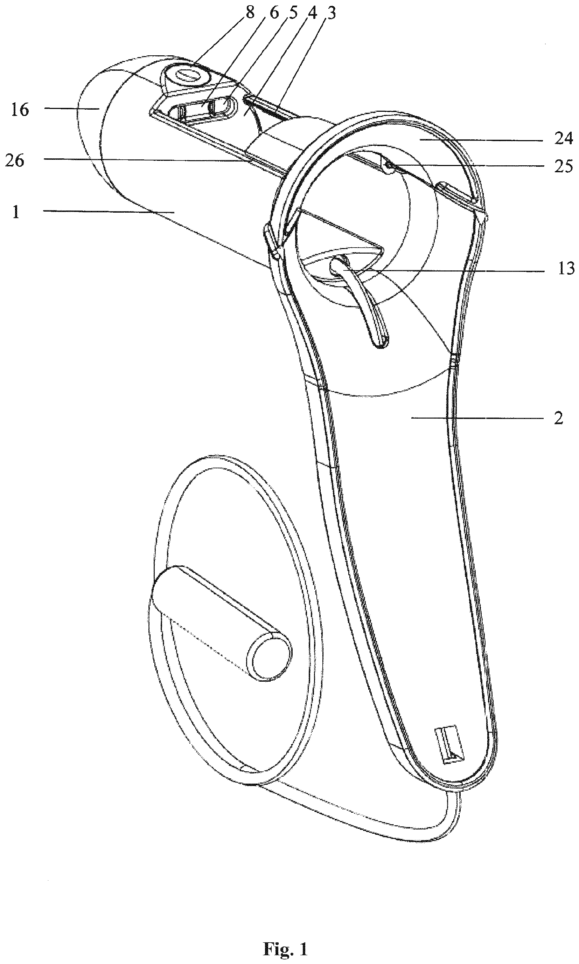

FIG. 1 is a perspective view of the proctoscope;

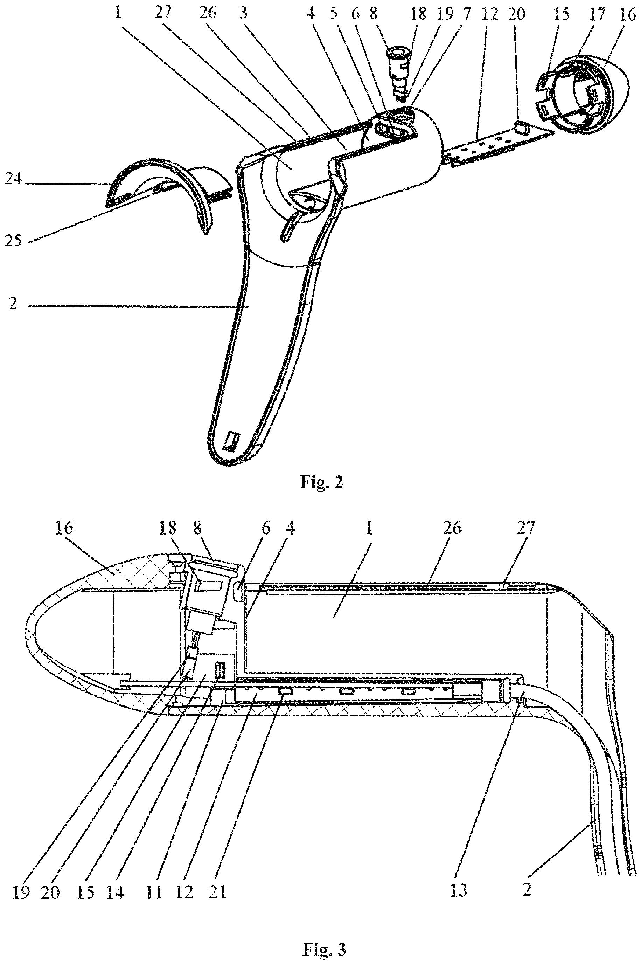

FIG. 2 is an exploded perspective view of the proctoscope;
FIG. 3 is a sectional view of the proctoscope taken along the longitudinal axis;

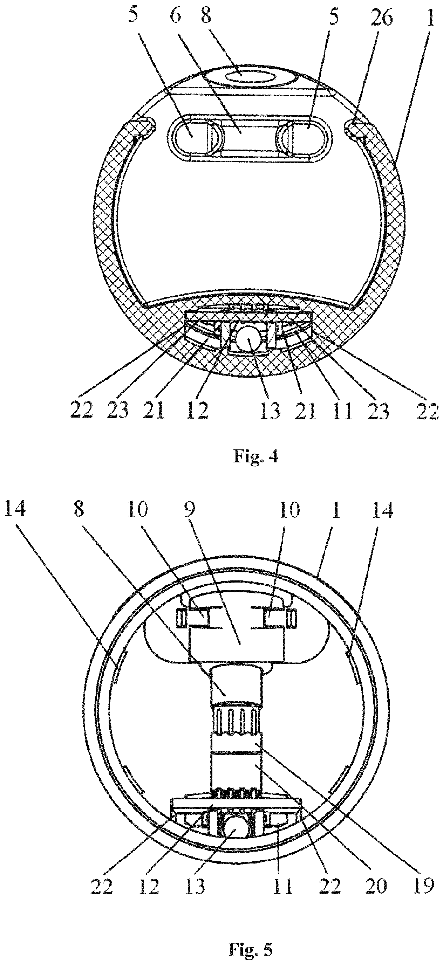

FIG. 4 shows a partition wall of the proctoscope in a cross-section perpendicular to the longitudinal axis, in a view in the direction of a housing head;
FIG. 5 is a cross-sectional view of the proctoscope from the side of the housing head (with the head removed and with an extension and a hand grip not shown).

### DETAILED DESCRIPTION

The proctoscope consists of a main body constituting the housing 1 in the form of a rounded barrel with a proximal portion expanding and smoothly blending into a flat hand grip (or a handle) 2, the main body being provided with an opening 3 for access to a rectum surface, the housing head, an ultrasonic (US) sensor, a light source.

The lateral surface of the housing has a cut in one of its sides formed by a plane parallel to the longitudinal axis of the housing, the cut resulting in the above-mentioned opening 3 for access to the rectum surface. The opening 3 is closed at the terminal (distal) part of the housing and expanding and open in the proximal part thereof. At its distal part, the opening 3 is closed by a blind perpendicular wall 4 having two rounded recesses 5 located closer to the upper border of the opening 3, interconnected by a groove 6 and intended for supporting a surgical instrument, in particular a needle holder.

Use of the recesses interconnected by the groove as supports for the medical instrument makes it possible to reduce a risk of injuring a mucous membrane by a needle when performing suturing owing to reliable fixation (by abutting) of the needle holder in the left (right) recess when making a first piercing of a mucosa by the needle followed by a smooth controlled movement of a tip ("a nose") of the needle holder in the groove while simultaneously turning the needle holder until the tip of the needle is withdrawn from the mucosa, which occurs when the nose of the needle holder is already located in the other, that is in the right (left) recess.

Distally of the wall 4, the lateral surface of the housing 1 is truncated by a plane facing the proximal part of the proctoscope, in which plane an aperture 7 is formed, said aperture being connected with a hollow cylinder 9 having through slots 10 for fixing the US sensor 8. In the lateral surface of the housing opposite to the truncated surface, a flattened cavity (serving as a cable channel) 11 is formed. The cavity 11 extends from the distal part of the housing 1 to its proximal extension, and is designed to accommodate a light source 12 and a cable 13 of the proctoscope. On the inner surface of the distal part of the housing, there are provided guides formed as projecting abutments 14 for snap latches 15 serving for locking a housing head 16, said head designed as a dome-shaped part, solid in its distal portion and open in its proximal portion, which part is manufactured separately from the proctoscope housing (or main body). Proctoscope housing is preferably made of a translucent material. Snap latches 15, adapted to lock together the head 16 and the housing 1 in the coaxial positions, are formed in side walls of the proximal portion of the head 16.

Bars 17, which are oriented proximally and in parallel to the head 16 axis of symmetry and abut the inner surface of the head, serve as stops adapted to fix the US sensor 8 inside the housing 1 by sliding into the through slots 10 and grooves 18 in the hollow cylinder when the head 16 is being connected to the housing 1.

The US sensor 8 is mounted inside the hollow cylinder 9 of the housing 1 and has, in its lateral surface, grooves 18 mating with corresponding slots 10 formed in the wall of the hollow cylinder to enable entering therein rods 17 of the head 16 in the course of assembling the proctoscope. In result of said assembly, an outer surface of the US sensor becomes a part of the outer surface of the housing. The US sensor is tilted toward the opening and has a connector 19 for connecting with a connector 20 of the light source 12.

By locating the US sensor outside the opening 3 for access to the rectum surface (outside the ligation area), more working space for manipulations with the medical instrument in the inner cavity of the proctoscope housing is freed.

The light source 12 has at least two LEDs 21 and is mounted in the bottom part of the housing 1, namely in a flattened compartment 11 with internal side walls 22. Inside the compartment 11 a plate or panel 23 is provided, with LEDs 21 mounted at the bottom part of said plate along its length, that is along the longitudal axis of the compartment 11. As a result, the LEDs emit light mainly in a direction perpendicular to the side walls 22, so the light spreads in the housing cavity and thus illuminates the entire space inside the housing and also the window 3 for access to the rectum surface. The plate or panel 23 serves also as a protective shield to minimize an amount of light entering directly the inner space of the proctoscope housing and to avoid, in this way, a possible blinding of a surgeon. The terminal (distal) part of the plate or panel 23 is positioned in abutment inside the head.

The use of the proposed embodiment of the light source results in generating, by multiple LEDs, a distributed light flux which propagates inside the walls of the entire housing of the proctoscope, so that significantly better illumination of the operation area is provided.

Proctoscope may comprise a removable insert 24. The insert is mounted within a proximal part of the housing 1 by means of U-shaped skids 25, mating with the guides 26 formed in the side of the housing. After mounting, the removable insert 24 is securely locked in the housing 1 (by means of locks formed by notches 27 in guides 26 and by mating protrusions formed in skids 25 of the removable insert) partially overlapping the opening 3 formed in the housing.

In case the removable insert is installed, it limits haemorrhoids prolapse into the opening for assess to the rectum surface. The removable insert is mainly used at the stage of arteries ligation.

If the removable insert is not installed, the device provides a free access to mucosa and to haemorrhoids, so that the stage of mucopexy (suturing mucosa) can be performed following the ligation stage.

Owing to the use of the stops 14 and the latches 15 supplied with annular gaskets (not shown in the drawings) and the connectors 19, 20 of the US sensor and the light source, the proctoscope can be quickly and effectively assembled as a modular construction.

Alternatively, the head 16 and the US sensor 8 can be fixed to the housing 1 by gluing, i. e. without any locks.

The US sensor 8 can be connected directly to the light source 12, that is without using the connector 20.

Anatomical studies have revealed that the main causative factors of haemorrhoids consist in pathological arterial inflow at the distal branches of the superior rectal artery (*a. rectalis superior),* as well as in degeneration and weakening of the ligamentous apparatus. This leads to hypertrophy of the cavernous tissue, bleeding and haemorrhoid prolapse from the anal orifice.

The inventive device is used in implementing a minimally invasive method of treating haemorrhoids, which method is based on the detection of haemorrhoidal arteries using the Doppler ultrasonography and subsequent transanal dearterialization of internal haemorrhoids under the Doppler ultrasonography control to stop or reduce the inflow of arterial blood supply to the haemorrhoid followed by mucopexy of mucosa and cavernous tissue in the anal canal.

In conducting the above-mentioned procedure, the device is introduced into the anal canal with holding the device by the hand grip and the expanded part of the housing. The initial position of the device in the anal canal is usually with hand grip down (and with the opening for access to the rectum surface at 12 o'clock position).

By progressively turning the device, a search for branches of the superior rectal artery to be ligated is carried out. The search is performed at a depth of 3-4 cm above the dentate line, with priority directions corresponding to 1, 3, 5, 7, 9, 11 o'clock positions and with using the US sensor mounted in the device and connected to the electronic unit converting signals from the sensor to appropriate visual and audio signals.

Then the medical instrument (the needle holder) with a rounded needle 5/8 is installed in the device. In case the device is formed with depressions or recesses for medical instruments, a nose of the needle holder is placed in one of such recesses formed in the wall of the distal portion of the housing.

An 8-shaped stitching of the mucosa and a haemorrhoidal artery found when performing the search is conducted by successively injecting the needle, turning it, moving the nose of the needle holder along the corresponding groove to another recess and continuing the rotation of the needle until its tip moves out of the mucosa.

After that, the needle is taken out from the mucosa, the nose of the needle holder is pulled from the recess, the tip of the needle is captured by the needle holder, the nose of the needle holder is thrusted against the nearest recess, the turn of the needle is completed, and the thread is pulled up.

Then the second injection with an appropriate offset and a similar circular rotation of the needle are carried out.

In order to successfully complete the ligation, a check is performed whether artery pulsation disappeared or is significantly reduced, and, after that, a knot is tied, with holding it by a special thread holder.

In case of haemorrhoid tissue prolapsing into the opening for access to the rectum surface (the so-called ligature window) of the device, after artery ligation is completed, mucopexy is performed by employing continuous suture with capturing of the cavernous tissue up to the dentate line, followed by pulling the tread and tying the knot.

The search for the upper rectal artery branches to be ligated is continued along a rectum circumference, and the above-described manipulations are repeated.

The operation principle of the US sensor (which constitute the transceiver component of the device) is based on the Doppler effect according to which the low-frequency component of the Doppler shift is linearly dependent on the relative velocity of objects (in this case of a stationary sensor and blood particles in a blood vessel). If an angle between a direction of blood flow in the vessel and a direction of the ultrasonic beam axis (sensor axis) is different from zero, the velocity vector is decomposed into orthogonal projections. When the above angle is close to 90 degrees, the projection of the velocity vector on the sensor axis is, respectively, very small. This means that the Doppler shift differential frequency is close to zero. Any medical electronic instrument necessarily comprises a high pass filter to cut off low-frequency Doppler signals from oscillating vessel walls and from mucosal and other tissues, the level of which signals exceeds, by thousands of times, a level of a signal reflected from the blood particles. Therefore, it is very difficult to record and process the "useful" Doppler signal in the frequency range below the specified cutoff. Considering that, in our case, the anatomical angle is close to 90 degrees, it is necessary to reduce the angle between the sensor axis and the vessel. This object is attained by tilting the sensor.

When the sensor is tilted in the direction of the opening for access to the rectum surface (towards the ligation zone and the plane in which the needle moves when clasping the vessel and "tying it off" with the tread so that the blood flow to the vessel is completely cut), we are bringing the vessel location zone of the sensor closer to the zone to be ligated and raise thereby accuracy of locating the vessel and reliability of stopping or substantially reducing the blood flow to the vessel.

## Claims

1. A proctoscope comprising: a translucent housing (1) having an opening (3) for access to the rectum surface; and a housing head (16); an ultrasonic sensor (8); a light source (12); and a hand grip (2); **characterized in that** the proctoscope includes, in a lower part of the housing, a compartment (11) having internal side walls (22), said compartment having disposed therein a plate or panel (23) which has light-emitting diodes arranged in the lower part thereof, along its length, and functioning as the light source (12), wherein said light-emitting diodes are arranged in such a way that the light flux therefrom is oriented primarily perpendicularly to the internal side walls of the compartment, while the ultrasonic sensor (8) is accommodated in an upper part of said plate or panel (23), said sensor (8) having an outlet to the outer surface of the housing in the terminal part thereof located between the opening (3) and the housing head (16), and wherein the sensor (8) is tilted towards the opening.

2. The proctoscope according to claim 1, **characterized in that** the plate or panel is adapted to serve as a shield providing protection from a part of the light flux generated by the light-emitting diodes.

3. The proctoscope according to claim 1, **characterized in that** the terminal part of the housing is divided by a partition wall from the remaining part of the housing.

4. The proctoscope according to claim 3, **characterized in that** the partition wall has a bulge in its upper part, said bulge serving, on one side of said wall, as a support for the ultrasonic sensor, wherein recesses serving as supports for a medical instrument are provided in the opposite side of said wall.

5. The proctoscope according to claim 3, **characterized in that** two rounded recesses are formed in said wall, said recesses being connected by a groove to ensure support for a medical instrument.

6. The proctoscope according to claim 1, **characterized in that** said head is made removable and is supplied with projecting guides having snap latches for fixedly attaching the head to the housing.

7. The proctoscope according to claim 1, **characterized in that** the head is provided with projecting stops for fixedly attaching the ultrasonic sensor.

8. The proctoscope according to claim 1, **characterized in that** it further comprises a removable insert supplied with skids and with locks and fixedly positioned in the housing partially overlapping said opening.

9. The proctoscope according to claim 1, **characterized in that** the terminal part of the plate or panel is positioned in abutment inside the head.

## Patentansprüche

1. Ein Rektoskop, aufweisend: ein lichtdurchlässiges Gehäuse (1) mit einer Öffnung (3) für einen Zugang zu der Rektumoberfläche, und einen Gehäusekopf (16), einen Ultraschallsensor (8), eine Lichtquelle (12) und einen Handgriff (2),
**dadurch gekennzeichnet, dass** das Rektoskop in einem unteren Teil des Gehäuses eine Kammer (11) mit inneren Seitenwänden (22) aufweist, wobei in der Kammer eine Platte oder ein Paneel (23) angeordnet ist, die/das lichtemittierende Dioden aufweist, die im unteren Teil davon entlang ihrer/seiner Länge angeordnet sind und als Lichtquelle (12) dienen, wobei die lichtemittierenden Dioden derart angeordnet sind, dass der Lichtstrom von diesen im Wesentlichen senkrecht zu den inneren Seitenwänden der Kammer ausgerichtet ist, während der Ultraschallsensor (8) in einem oberen Teil der Platte oder des Paneels (23) untergebracht ist, wobei der Sensor (8) einen Auslass zu der Außenfläche des Gehäuses im Endteil davon aufweist, der zwischen der Öffnung (3) und dem Gehäusekopf (16) angeordnet ist, und wobei der Sensor (8) in Richtung zu der Öffnung geneigt ist.

2. Das Rektoskop gemäß Anspruch 1, **dadurch gekennzeichnet, dass** die Platte oder das Paneel angepasst ist, um als eine Abschirmung zu dienen, die Schutz vor einem Teil des von den lichtemittierenden Dioden erzeugten Lichtstroms bietet.

3. Das Rektoskop gemäß Anspruch 1, **dadurch gekennzeichnet, dass** der Endteil des Gehäuses durch eine Trennwand vom restlichen Teil des Gehäuses getrennt ist.

4. Das Rektoskop gemäß Anspruch 3, **dadurch gekennzeichnet, dass** die Trennwand in ihrem oberen Teil eine Ausbuchtung hat, wobei die Ausbuchtung auf einer Seite der Wand als eine Abstützung für den Ultraschallsensor dient, wobei Aussparungen, die als Abstützungen für ein medizinisches Instrument dienen, in der entgegengesetzten Seite der Wand vorgesehen sind.

5. Das Rektoskop gemäß Anspruch 3, **dadurch gekennzeichnet, dass** zwei abgerundete Aussparungen in der Wand ausgebildet sind, wobei die Aussparungen durch eine Nut verbunden sind, um eine Stütze für ein medizinisches Instrument zu gewährleisten.

6. Das Rektoskop gemäß Anspruch 1, **dadurch gekennzeichnet, dass** der Kopf abnehmbar ausgebildet ist und mit hervorstehenden Führungen mit Schnappverschlüssen für ein festes Anbringen des Kopfes am Gehäuse versehen ist.

7. Das Rektoskop gemäß Anspruch 1, **dadurch gekennzeichnet, dass** der Kopf mit hervorstehenden Anschlägen zum festen Anbringen des Ultraschallsensors versehen ist.

8. Das Rektoskop gemäß Anspruch 1, **dadurch gekennzeichnet, dass** es ferner einen abnehmbaren Einsatz aufweist, der mit Gleitschienen und mit Verriegelungen versehen ist und fest in dem Gehäuse positioniert ist, wobei er die Öffnung teilweise überlappt.

9. Das Rektoskop gemäß Anspruch 1, **dadurch gekennzeichnet, dass** der Endteil der Platte oder des Paneels innerhalb des Kopfes angrenzend positioniert ist.

## Revendications

1. Rectoscope comprenant : un boîtier translucide (1) ayant une ouverture (3) pour avoir accès à la surface rectale ; et une tête de boîtier (16) ; un capteur ultrasonore (8) ; une source de lumière (12) ; et une poignée (2) ;
**caractérisé en ce que** le rectoscope comprend, dans une partie inférieure du boîtier, un compartiment (11) ayant des parois latérales internes (22), ledit compartiment ayant, disposé(e) à l'intérieur de ce dernier, une plaque ou un panneau (23) qui a des diodes électroluminescentes agencées dans sa partie inférieure, le long de sa longueur, et servant de source de lumière (12), dans lequel :
lesdites diodes électroluminescentes sont agencées de sorte que le flux lumineux à partir de ces dernières est principalement orienté perpendiculairement aux parois latérales internes du compartiment, alors que le capteur ultrasonore (8) est logé dans une partie supérieure de ladite plaque ou dudit panneau (23), ledit capteur (8) ayant une sortie sur la surface externe du boîtier dans sa partie terminale positionnée entre l'ouverture (3) et la tête de boîtier (16),
et dans lequel le capteur (8) est incliné vers l'ouverture.

2. Rectoscope selon la revendication 1, **caractérisé en ce que** la plaque ou le panneau est adapté pour servir de protection fournissant la protection par rapport à une partie du flux lumineux généré par les diodes électroluminescentes.

3. Rectoscope selon la revendication 1, **caractérisé en ce que** la partie terminale du boîtier est divisée par une paroi de séparation par rapport à la partie résiduelle du boîtier.

4. Rectoscope selon la revendication 3, **caractérisé en ce que** la paroi de séparation a un renflement dans sa partie supérieure, ledit renflement servant, sur un côté de ladite paroi, de support pour le capteur ultrasonore, dans lequel les évidements servant de supports pour un instrument médical sont prévus dans le côté opposé de ladite paroi.

5. Rectoscope selon la revendication 3, **caractérisé en ce que** deux évidements arrondis sont formés dans ladite paroi, lesdits évidements étant raccordés par une rainure pour garantir le support pour un instrument médical.

6. Rectoscope selon la revendication 1, **caractérisé en ce que** ladite tête est rendue amovible et est alimentée avec des guides en saillie ayant des verrous d'encliquetage pour fixer de manière fixe la tête sur le boîtier.

7. Rectoscope selon la revendication 1, **caractérisé en ce que** la tête est prévue avec des butées en saillie pour fixer, de manière fixe, le capteur ultrasonore.

8. Rectoscope selon la revendication 1, **caractérisé en ce qu'**il comprend en outre un insert amovible doté de patins et de verrous et positionné de manière fixe dans le boîtier chevauchant partiellement ladite ouverture.

9. Rectoscope selon la revendication 1, **caractérisé en ce que** la partie terminale de la plaque ou du panneau est positionnée en butée à l'intérieur de la tête.
